# EUROPEAN PATENT SPECIFICATION

(11) **EP 2 702 982 B1**
(45) Date of publication and mention of the grant of the patent: **14.12.2016**
(21) Application number: 12776642.6
(22) Date of filing: 25.04.2012
(51) Int. Cl.: A61K 8/85, A61K 8/49, A61K 8/64, A61Q 7/00, A61K 31/506, A61K 31/565, A61K 31/58, A61K 9/00

(54) **COMPOSITION FOR TOPICAL APPLICATION FOR PREVENTING HAIR LOSS AND STIMULATING HAIR GROWTH**
TOPISCH ANGEWENDETE ZUSAMMENSETZUNG ZUR VORBEUGUNG VON HAARAUSFALL UND ZUR STIMULIERUNG DES HAARWACHSTUMS
COMPOSITION À APPLIQUER PAR VOIE TOPIQUE DESTINÉE À PRÉVENIR LA CHUTE DES CHEVEUX ET À STIMULER LEUR CROISSANCE

(30) Priority: 25.04.2011 KR 20110038660; 05.08.2011 US 201161515523 P
(43) Date of publication of application: 05.03.2014
(73) Proprietor: Park, Jun-Hyoung, Busan 612-823 (KR)
(72) Inventor: Park, Jun-Hyoung, Busan 612-823 (KR)
(74) Representative: Prüfer & Partner mbB Patentanwälte · Rechtsanwälte
(86) International application number: PCT/KR2012/003200
(87) International publication number: WO 2012/148174

(56) References cited:
- WO-A1-00/66072
- KR-A- 20050 119 525
- KR-A- 20050 119 525
- KR-A- 20090 010 178
- KR-B1- 100 858 224
- US-A1- 2006 052 405
- DIANI A R ET AL: "HAIR GROWTH EFFECTS OF OREAL ADMINISTRATION OF FINASTERIDE, A STEROID 5ALPHA-REDUCTASE INHIBITOR, ALONE AND IN COMBINATION WITH TOPICAL MINOXIDIL IN THE BALDING STUMPTAIL MACAQUE", JOURNAL OF CLINICAL ENDOCRINOLOGY AND METABOLISM, THE ENDOCRINE SOCIETY, US, vol. 74, no. 2, 1 February 1992 (1992-02-01), pages 345-350, XP000604902, ISSN: 0021-972X, DOI: 10.1210/JC.74.2.345
- JAVADZADEH YOUSEF ET AL: "Enhancement of percutaneous absorption of finasteride by cosolvents, cosurfactant and surfactants.", December 2010 (2010-12), PHARMACEUTICAL DEVELOPMENT AND TECHNOLOGY DEC 2010, VOL. 15, NR. 6, PAGE(S) 619 - 625, XP9186127, ISSN: 1097-9867

## Description

### [Technical Field]

The present invention relates to a composition for use in topical application for treating alopecia,said composition comprising a 5α-reductase inhibitor and minoxidil.

### [Background Art]

According to the studies conducted so far, it has been found that the causes of hair loss include endocrine system disorders such as hormonal imbalance, etc., excessive sebum formation caused by circulatory system disorders such as autonomic nervous system disorders, blood circulation disorders, etc., nutritional deficiency of hair roots, allergies, bacterial infections, genetic factors, psychological stress, environmental factors such as atmospheric pollution or foods, and aging, etc. In the past, alopecia occurred only in the middle-aged and elderly, but recently, alopecia also has occurred frequently in young people aged 20-30 years and in women.

The effects of products which are sold as hair growth stimulating-agents or hair loss preventing-agents on hair include growth period inducing effects, hair growth period extending effects, 5α-reductase inhibitory effects, blood circulation promoting effects, antiseptic effects, antidandruff effects, moisturizing effects, antioxidant effects, etc., but the effects of preventing hair loss and stimulating hair growth of conventional agents are not sufficient.

Male pattern alopecia is dependent on male hormones and is thus directly related to the amount of male hormones. Therefore, extensive research aimed at preventing and treating hair loss by inhibiting the activity of male hormones has recently been reported. The mechanism of hair loss related to male hormones is briefly described below. That is, testosterone, which is one of the male hormones, is converted into dihydrotestosterone, which is an active male hormone, by a 5α-reductase, and the active male hormone binds to a specific receptor to produce a protein that triggers hair loss, thus causing hair loss. Also, this mechanism produces excessive sebum, which causes acne or seborrheic dermatitis, eventually causing hair loss accompanied by inflammation in the scalp. As a result, the cause of male pattern alopecia is the synthesis of excessive dihydrotestosterone by the action of the 5α-reductase, and thus it is possible to fundamentally and effectively prevent and treat male pattern alopecia by inhibiting the activity of the 5α-reductase.

Currently, the most commonly used drugs for the treatment of hair loss include a topical formulation comprising 2,4-diamino-6-Piperidinopyrimidin-3-oxide (also known as "Minoxidil" formulation, see U.S. Patent Nos. 4,139,619 and4,596,812), which has been approved by the FDA, and an oral formulation comprising finasteride, which is a specific inhibitor of type II 5α-reductase.

The minoxidil was developed for the purpose of lowering blood pressure of hypertensive patients, but it is most widely used as a hair growth drug since its use changed due to hair growth side effects that occurred during the use. The action mechanism of minoxidil that was approved for use as a hair loss treatment agent by the U.S. Food and Drug Administration (FDA) in 1979 has not been clearly elucidated. However, the action mechanism of minoxidil has been explained by a hypothesis that the minoxidil increases blood flow to the follicles to cause an increase in blood flow, thus stimulating the growth of hair, and a hypothesis that the minoxidil acts directly on the follicular epithelium to induce the growth of hair. However, hair restorers comprising minoxidil should be applied several times daily to maintain the hair growth effect, which is very cumbersome and easy to forget. Therefore, in many cases, the hair growth effect is not sufficiently obtained due to irregular application and arbitrary discontinuation of treatment.

Moreover, based on the mechanism of action of finasteride on male hormones, there is a method of preventing hair from becoming thin and of making hair thicker by antagonism of finasteride on the 5α-reductase. Representatively, there is Propecia that was developed by Merck & Co. Inc., approved by the U.S. Food and Drug Administration (FDA) in December 1997 for the efficacy and safety as a hair loss treatment drug, and commercially released in 1998. Finasteride is a drug that inhibits the 5α-reductase which converts testosterone, one of the male hormones, into dihydrotestosterone (DHT) that causes hair loss. The administration of the drug inhibits the production of dihydrotestosterone, and thus the finasteride allows fine hair in bald areas to grow thick and long. As a result of clinical trials conducted on 1,879 men at the time of development, only 17% of the participants who were treated constantly for two years showed no hair loss, and 83% of the participants maintained or increased the number of hairs after the treatment. However, it takes several months to obtain the effect of preventing hair loss. Moreover, in case of females, there is a high risk of congenital deformity in fetus, and in the case of males, there are fears of side effects such as hyposexuality, impotence, ejaculation disorder, etc. Further, the pressure to take up the drug during life so as to maintain the effect preventing hair loss causes significant restrictions in actual clinical use.

Meanwhile, the U.S. Food and Drug Administration (FDA) has recently amended its approval regarding the duration of possible sexual side effects of a drug containing 1 mg of finasteride, which is used for the treatment of male pattern alopecia, even after discontinuation of use, and recommended attention to medical professionals and patients. As a result of reviewing the postmarketing cases reported on the FDA's Adverse Event Reporting System (AERS) and safety databases of marketed products, it was found that some sexual function-related adverse reactions (such as hyposexuality, ejaculation disorder, orgasmic disorder, etc.) continued even after discontinuation of use. Moreover, it was revealed that that the causal relationship between the drug containing the ingredient in question and the sexual side effects was not clearly established.

### [Disclosure]

### [Technical Problem]

The present invention has been made to solve the above problems of the prior art, and an object of the present invention is to provide a composition for topical application for preventing hair loss and stimulating hair growth, which has the following advantages:
(1) It provides the effects of preventing hair loss and stimulating hair growth that are equal to or higher than that of conventional treatment agents even though the amount of a 5α-reductase inhibitor used is less than one quarter that of the conventional treatment agents;
(2) There are almost no side effects of the conventional treatment agents;
(3) It is possible to effectively prevent hair loss from the beginning of treatment due to a rapid onset of the effect; and
(4) It provides almost 100% effect to patients with hair loss, unlike the conventional prescription that provides about 70% effect.

### [Technical Solution]

To achieve the above objects, the present invention provides a composition for topical application for preventing hair loss and stimulating hair growth, containing a 5α-reductase inhibitor, and minoxidil or a pharmaceutically acceptable salt thereof.

More specifically, the present invention provides a composition for topical application for preventing hair loss and stimulating hair growth, comprising: finasteride, dutasteride, or a pharmaceutically acceptable salt thereof; and minoxidil or a pharmaceutically acceptable salt thereof.

### [Advantageous Effects]

The composition for topical application for preventing hair loss and stimulating hair growth of the present invention provides the effects of preventing hair loss and stimulating hair growth that are equal to or higher than that of conventional treatment agents even though the amount of a 5α-reductase inhibitor used is much less (less than one quarter) that of a conventional oral 5α-reductase inhibitor.

Moreover, it uses a small amount of drugs and is in a dosage form for topical application, and thus it has little side effects such as hyposexuality, impotence, ejaculation disorder, etc. Further, it can effectively prevent hair loss from the beginning of treatment due to a fast onset of the effect than the use of a conventional oral 5α-reductase and minoxidil alone or together and can provide the effect of improving treatment compliance of patients. Furthermore, it provides almost 100% effect to hair loss patients, unlike the conventional prescription that is effective only for about 70% of hair loss patients.

### [Best Mode]

Hereinafter, the present invention will be described in detail.

The present invention relates to a composition for topical application for preventing hair loss and stimulating hair growth, comprising a 5α-reductase inhibitor, and minoxidil or a pharmaceutically acceptable salt thereof.

The daily dose of the 5α-reductase inhibitor contained in the topical application for preventing hair loss and stimulating hair growth and transdermally administered is 0.8 µg to 0.12 mg, which is about less than one quarter that of oral dutasteride (0.5 mg), a current commercially available 5α-reductase inhibitor (the daily dose of commercially available oral finasteride is 1 mg).

More preferably, the daily dose of the 5α-reductase inhibitor is 0.8 µg to 0.05 mg, and most preferably, the daily dose of the 5α-reductase inhibitor is 0.001 mg to 0.025 mg.

If the daily dose of the 5α-reductase inhibitor is less than 0.8 µg, the onset of the effect is insignificant, whereas, if it exceeds 0.12 mg, side effects such as hyposexuality, ejaculation decrease, etc. may occur.

The daily dose of minoxidil or a pharmaceutically acceptable salt thereof contained in the topical application for preventing hair loss and stimulating hair growth and transdermally administered is 0.008 g to 0.12 g. If the daily dose is less than 0.008 g, the desired effect cannot be obtained, whereas if it exceeds 0.12 g, side effects such as increased cardiac output, increased left ventricular end-diastolic volume, etc. may occur.

If the 5α-reductase inhibitor is finasteride or a pharmaceutically acceptable salt thereof, the daily dose may preferably be 0.001 mg to 0.12 mg, more preferably 0.005 mg to 0.05 mg, and most preferably 0.005 mg to 0.025 mg. If the dose of finasteride or a pharmaceutically acceptable salt thereof is out of the range, the effect is insufficient or side effects may occur.

If the 5α-reductase inhibitor is dutasteride or a pharmaceutically acceptable salt thereof, the daily dose may preferably be 0.8 µg to 0.06 mg, more preferably 0.0016 mg to 0.025 mg, and most preferably 0.0016 mg to 0.012 mg. If the dose of dutasteride or a pharmaceutically acceptable salt thereof is out of the range, the effect is insufficient or side effects may occur.

The 5α-reductase inhibitor has side effects such as hyposexuality, impotence, ejaculation disorder, etc., and the FDA has recently amended its approval regarding the duration of these side effects even after discontinuation of use and recommended attention to medical professionals and patients.

Therefore, it is required to reduce the dose of the 5α-reductase inhibitor so as to reduce these side effects. Therefore, extensive research aimed at allowing the 5α-reductase inhibitor to reach an accurate target by oral administration has been actively conducted. Meanwhile, an attempt to transdermally administer finasteride, a 5α-reductase inhibitor, has also been made, but no significant effect was obtained.

The composition for topical application of the present invention, in which the 5α-reductase inhibitor and minoxidil are dissolved in solvent, is transdermally applied to a target such that the 5α-reductase inhibitor is very effectively delivered to the target by syniergistic effect, thus (1)providing almost 100% effect to hair loss patients, and (2) providing very rapid and excellent effects even though the amount of the 5α-reductase inhibitor used is less than one quarter that of the conventional prescription.

It is considered that the above significant effect results from the transdermal delivery efficiency of the 5α-reductase inhibitor that is significantly improved by vasodilation effect of minoxidil.

The 5α-reductase (reduced nicotinamide adenine dinucleotide phosphate:Δ4-3-ketosteroid 5α-oxidoreductase) is present in type I and type II isozymes and stimulates the reductive conversion of testosterone into 5α-dihydrotestosterone. Testosterone mainly plays a role in determining the morphology of the male internal genitalia, and dihydrotestosterone is required for development of the external genitalia.

The type I 5α-reductase is mainly distributed around sebaceous glands and the type II 5α-reductase is found in the dermal papillae, which are crucial for follicle growth, and in the outer root sheath of follicles. Dihydrotestosterone (DHT) produced by the 5α-reductase binds to receptors of follicular cells, and the DHT-receptor complex enters cell nucleus so that the DNA induces the synthesis of various protein regulators that influence the proliferation and differentiation of follicular cells. These various regulators formed by DHT stimulate the proliferation of hair matrix cells to accelerate the growth period (growth stage 1-growth stage 5) to relatively shorten the differentiation period (growth stage 6-regression stage) of follicular cells, thus increasing hair loss and inhibiting hair growth.

It is preferable that the 5α-reductase inhibitor is finasteride, dutasteride, or a pharmaceutically acceptable salt thereof.

The finasteride inhibits only the type I isozyme 5α-reductase, which is an enzyme that converts testosterone into dihydrotestosterone (DHT), and the dutasteride inhibits both the type I and type II isozymes of 5α-reductase to strongly maintain testosterone, thus inhibiting hair loss.

The structure of finasteride is represented by the following formula 1.

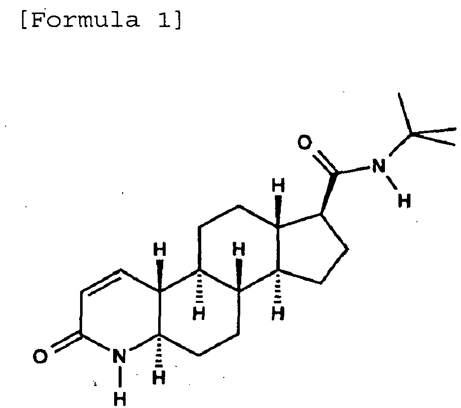

The structure of dutasteride is represented by the following formula 2.

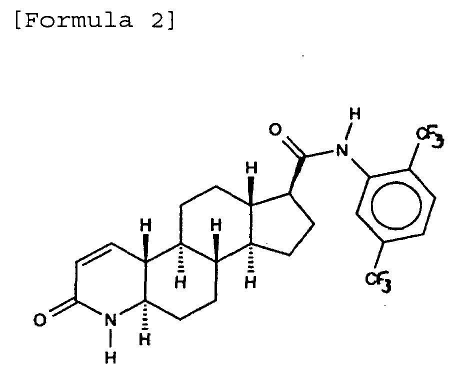

The minoxidil, a lipophilic ingredient, is the only topical application approved by the FDA as a hair restorer and treatment agent for female pattern hair loss that has an excellent ability to penetrate into hair roots and serves to supply nutrients useful for hair growth by improving blood flow around the follicles of the scalp.

The structure of minoxidil is represented by the following formula 3.

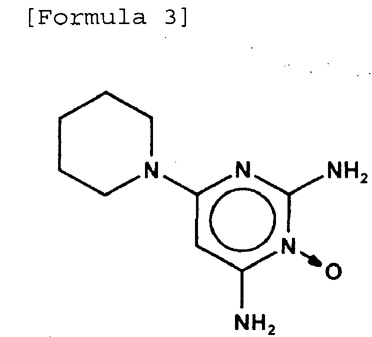

The composition for topical application for preventing hair loss and stimulating hair growth may be prepared, comprising finasteride or a pharmaceutically acceptable salt thereof in an amount of 0.1 mg to 5 mg, more preferably 0.1 mg to 2 mg with respect to 100 ml of the composition for topical application. Also, it may be prepared, comprising minoxidil or a pharmaceutically acceptable salt thereof in an amount of 0.5 g to 5 g respect to 100 ml of the composition for topical application.

Moreover, the composition for topical application for preventing hair loss and stimulating hair growth may be prepared, comprising dutasteride or a pharmaceutically acceptable salt thereof in an amount of 0.05 mg to 2.5 mg, preferably 0.1 mg to 1.0 mg with respect to 100 ml of the composition for topical application. Also, it may be prepared, comprising minoxidil in an amount of 0.5 g to 5 g respect to 100 ml of the composition for topical application.

It is preferable that the composition for topical application prepared in the above manner is applied in an amount of 100 ml once or twice a day for 45 to 60 days.

The finasteride, dutasteride, and minoxidil of the present invention may be used in the form of pharmaceutically acceptable salts, and acid addition salts formed with pharmaceutically acceptable free acids are useful as such salts. Acid addition salts are obtained from inorganic acids such as hydrochloric acid, nitric acid, phosphoric acid, sulfuric acid, hydrobromic acid, hydriodic acid, nitrous acid, or phosphorous acid, and non-toxic organic acids such as aliphatic mono- and dicarboxylate, phenyl-substituted alkanoate, hydroxy alkanoate and alkandioate, aromatic acids, and aliphatic and aromatic sulfonic acids. Such pharmaceutically non-toxic salts include sulfate, pyrosulfate, bisulfate, sulfite, bisulfite, nitrate, phosphate, monohydrogen phosphate, dihydrogen phosphate, metaphosphate, pyrophosphate chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maliate, butin-1,4-dioate, hexane-1,6-dioate, benzoate, chlorobenzoate, methyl benzoate, dinitrobenzoate, hydroxybenzoate, methoxybenzoate, phthalate, terephthalate, benzene sulfonate, toluene sulfonate, chlorobenzene sulfonate, xylene sulfonate, phenyl acetate, phenyl propionate, phenyl butyrate, citrate, lactate, β-hydroxybutyrate, glycolate, malate, tartrate, methane sulfonate, propane sulfonate, naphthalene-1-sulfonate, naphthalene-2-sulfonate, or mandelate, but not limited thereto.

The composition for topical application for preventing hair loss and stimulating hair growth for women may further comprise estradiol, preferably alfatradiol (17α-estradiol).

The alfatradiol, which is an estrogen derivative, may prevent testosterone from being converted into dihydrotestosterone and stimulate the proliferation of follicle cells. Particularly, the alfatradiol can be effectively used for the treatment of female hair loss patients.

Any solvents, which are typically used in the art, may be used as the solvent of the composition for topical application for preventing hair loss and stimulating hair growth, and the solvent may comprise at least one selected from group consisting of: water; a lower mono-alcohol having 1 to 4 carbon atoms including methanol, ethanol, isopropyl alcohol, and butanol; a polyhydric alcohol including ethylene glycol, propylene glycol, 1,3-butylene glycol; glycerin; and mixtures thereof.

Particularly, it is preferable that the solvent comprises a lower mono-alcohol having 1 to 4 carbon atoms, a polyhydric alcohol, and water for effective drug delivery.

In the foregoing, the lower mono-alcohol having 1 to 4 carbon atoms may preferably include, but not limited to, ethanol, and the polyhydric alcohol may preferably include, but not limited to, propylene glycol.

When the solvent comprises a lower mono-alcohol having 1 to 4 carbon atoms, a polyhydric alcohol, and water, it is preferable to contain 10 to 60 vol% lower mono- alcohol having 1 to 4 carbon atoms, 3 to 20 vol% polyhydric alcohol, and the remainder water, more preferably 30 to 50 vol% lower mono-alcohol having 1 to 4 carbon atoms, 5 to 15 vol% polyhydric alcohol, and the remainder water with respect to the total volume of the solvent.

When the solvent has the above composition ratio, the efficiency of transdermal drug delivery is significantly increased.

The composition for topical application for preventing hair loss and stimulating hair growth of the present invention may be prepared in any dosage forms such as liquid or gel type, which can be applied to the scalp. However, the composition may preferably be prepared in a dosage form selected from the group consisting of hair tonic, scalp treatment, hair cream, general ointment, cosmetic water, powder, essence, pack, hair dye, shampoo, conditioner, lotion, gel, patch, and spray type, more preferably a spray type or gel type.

If the amount of the ingredients such as finasteride, dutasteride, and minoxidil used increases, systemic absorption increases, and thus the possibility of occurrence of side effects may increase. The amount of finasteride, dutasteride, or minoxidil used in the present invention is much smaller than that of the conventional hair growth stimulating-agents, and the present invention can minimize side effects by selective administration through topical absorption and provide excellent effects of preventing hair loss and stimulating hair growth.

Moreover, while the treatment failure rate of conventional oral administration forms is high due to irregular administration, the method of applying the composition of the present invention in a spray type stimulates the effects of decreasing the risk of side effects, increasing the hair thickness, and reducing the hair loss in a short period of time, and is easy to facilitate the application, which in turn increases the compliance of patients, thus increasing the treatment success rate.

The composition for topical application for preventing hair loss and stimulating hair growth, containing finasteride and minoxidil, is mainly for men, and a composition for topical application containing finasteride and minoxidil of low concentration is also available for non-childbearing women.

The composition for topical application for preventing hair loss and stimulating hair growth, containing dutasteride and minoxidil, is available for men or non-childbearing women.

Moreover, the composition for topical application may further comprise an excipient typically used in the art, preferably a pharmaceutically acceptable excipient selected from the group consisting of a pH adjusting agent, a solubilizing agent, and a combination thereof within the range that the object and effect of the present invention are not impaired.

The pH adjusting agent may be selected from the group consisting of citric acid, malic acid, lactic acid, phosphoric acid, hydrochloric acid, sulfuric acid, and a combination thereof, but not limited thereto.

The solubilizing agent may be selected from the group consisting of diethylene glycol monoethyl ether, benzyl alcohol, glycerin, octoxynol, propylene glycol, propylene carbonate, polyethylene glycol, and a mixture thereof, but not limited thereto.

### [Mode for Invention]

Hereinafter, the present invention will be described in more detail with reference to Examples and Test Examples. However, it should be understood that the following Examples and Test Examples are provided only for illustrating the present invention in more detail, and the scope of the present invention is not limited by the following Examples and Test Examples.

### Examples 1 to 6: Preparation of composition for topical application for preventing hair loss and stimulating hair growth

After mixing ethanol, propylene glycol, and water at a volume ratio of 4:1:5, finasteride or dutasteride and minoxidil in amounts shown in Table 1 were dissolved in the mixed solution, thus preparing each 100 ml of a composition for topical application for preventing hair loss and stimulating hair growth in Examples 1 to 6.

**[Table 1]**

| Ingredient | Example 1 | Example 2 | Example 3 | Example 4 | Example 5 | Example 6 |
|---|---|---|---|---|---|---|
| Finasteride | 1 mg (0.001%) | 1 mg (0.001%) | 2 mg (0.002%) | 2 mg (0.002%) | - | - |
| Dutasteride | - | - | - | - | 0.2 mg (0.0002%) | 0.5 mg (0.0005%) |
| Minoxidil | 5 g (5%) | 2 g (2%) | 2 g (2%) | 5 g (5%) | 2 g (2%) | 2 g (2%) |

| | | | | | | |
|---|---|---|---|---|---|---|
| Note: % concentration in the present invention represents w/v % concentration. | | | | | | |

### Comparative Examples 1 to 3.

In Comparative Examples 1 to 3, commercially available finasteride oral medication, dutasteride oral medications, and minoxidil topical applications were purchased to prepare compositions comprising the oral medication and the topical medication together or a topical application alone using the following ingredients shown in the following Table 2.

**[Table 2]**

| Ingredient | Comparative Example 1 | Comparative Example 2 | Comparative Example 3 |
|---|---|---|---|
| Finasteride oral medication | 1 mg | - | |
| Dutasteride oral medication (mg) | - | 0.5 | |
| Minoxidil topical application (%) | 5% | 5% | 5% |

### Test Example 1: Clinical trials on effects of preventing hair loss and stimulating hair growth

### (1) Evaluation of changes in hair thickness and number

Each 100 ml of the compositions for topical application of Examples 1 to 6 was placed in a spray container and sprayed onto hair loss areas of alopecia patients twice a day (morning and evening), and then the effect was evaluated. Each 100 ml of the composition for topical application of Examples 1 to 6 was used for about 45 to 60 days, and the compositions were prepared by the same method as mentioned above upon completion of use. For the prescription of Comparative Examples 1 and 2, the oral medication was administered to alopecia patients once a day, and the minoxidil topical application was applied to hair loss areas and expected hair loss areas twice a day, and then the effect was evaluated. For the prescription of Comparative Example 3, only the minoxidil topical application was applied onto hair loss areas and expected hair loss areas of alopecia patients twice a day, and then the effect was evaluated.

The alopecia patients were selected according to the selection and exclusion criteria shown in the following Table 3, and when the patients first visited the hospital, their hair conditions were diagnosed, hair loss areas were marked and recorded, and black dots were tattooed on the most serious area so that the same area could be measured every time. Moreover, the number of hairs per unit area (hair number/cm²) and the hair thickness (µm) were measured using a simple hand-held phototrichogram (Folliscope, Lead M Co, Seoul, Korea). Hair loss area around the marked area was photographed using a 30 magnification lens, and then the number of terminal hairs per unit area was calculated. The thickness of hair was determined as the average thickness of 5 hairs around the marked area by measuring with 200 magnification lens.

The change in the number of hairs (hair number /cm²) and the change in the hair thickness (µm) in the alopecia patients were measured before administration and at a certain time after administration and shown in the following Tables 4, 5, and 7, and then the significance of these data was evaluated by T-TEST (including F-TEST). The results of T-TEST (including F-TEST) evaluation are shown in the Tables 6 and 8.

**[Table 3]**

| | |
|---|---|
| Alopecia patient selection criteria | (1) Male or female patients of 18 to 60 years old diagnosed as androgenetic alopecia; (2) Androgenetic alopecia patients with basic type diagnosed as M2 disorder, C2 disorder or U1 disorder and specific type diagnosed as V1 disorder or F1 disorder by Basic and specific(BASP) classification; (3) Patients who will not use any special hair product or undergo any hair care and treatment during the study period; (4) Patients who will maintain the same hair style during the study period; and (5) Male patients who are experiencing rapid hair loss recently |
| Alopecia patient exclusion criteria | (1) People with serious acute kidney or heart disease, or other chronic diseases (hypertension, diabetes, etc.)that may affect the test results in the past six months; (2) People with psychiatric diseases; (3) People with infectious skin diseases; (4) People who had surgical treatments for hair loss such as hair transplants, scalp reduction, etc.; (5) People who have used oral medication Finasteride or oral medication Dutasteride in the past 12 months; (6) People who have applicated any topical hair restorer, hair tonic, or hair growth promoter in the last six months; (7) People who are expected to have difficulties in application of test substance due to severe seborrheic dermatitis, scalp psoriasis, scalp infection, etc; (8)Patient with other hair loss diseases such as alopecia areata, telogen alopecia, cicatricial alopecia, etc. other than androgenetic alopecia; and (9)People who are expected to have difficulties to perform the test according to the opinion of the test director, other than the above items. |

**[Table 4]**

| | Composition ratio | | Measuring time | 2 weeks | 4 weeks | 6 weeks | 7 weeks | 8 weeks |
|---|---|---|---|---|---|---|---|---|
| Example 1 | Finasteride 0.001% + Minoxidil 5% | 1 | Increased number of hairs | | | 23 | | |
| | | | Increased hair thickness (µm) | | | 0.012 | | |
| | | 1 | Increased number of hairs | 29 | | | | |
| | | | Increased hair thickness (µm) | 0.002 | | | | |
| | | 2 | Increased number of hairs | | | | 26 | |
| | | | Increased hair thickness (µm) | | | | 0.012 | |
| | | 3 | Increased number of hairs | | | 19 | | |
| | | | Increased hair thickness (µm) | | | 0.009 | | |
| | | 4 | Increased number of hairs | | 13 | | | 16 |
| | | | Increased hair thickness (µm) | | 0.028 | | | 0.03 |
| | | 5 | Increased number of hairs | 4 | | | 9 | |
| Example 2 | Finasteride 0.001% + Minoxidil 2% | | Increased hair thickness (µm) | 0.006 | | | 0.012 | |
| | | 6 | Increased number of hairs | | 3 | | | 8 |
| | | | Increased hair thickness (µm) | | 0.003 | | | 0.004 |
| | | 7 | Increased number of hairs | | 1 | | | 9 |
| | | | Increased hair thickness (µm) | | 0.004 | | | 0.006 |
| | | 8 | Increased number of hairs | | 3 | | | 8 |
| | | | Increased hair thickness (µm) | | 0.003 | | | 0.004 |
| | | 9 | Increased number of hairs | | -5 | | | 6 |
| | | | Increased hair thickness (µm) | | 0.004 | | | 0.007 |
| | | 10 | Increased number of hairs | | 3 | | | 10 |
| | | | Increased hair thickness (µm) | | 0.003 | | | 0.011 |
| | | 11 | Increased number of hairs | | 11 | | | 19 |
| | | | Increased hair thickness (µm) | | 0.005 | | | 0.01 |
| Example 3 | Finasteride 0.002% + Minoxidil 2% | 1 | Increased number of hairs | | | 24 | | |
| | | | Increased hair thickness (µm) | | | 0.012 | | |
| | | 2 | Increased number of hairs | | | | | 35 |
| | | | Increased hair thickness (µm) | | | | | 0.021 |
| Comparative example 1 | Finasteride 1mg + Minoxidil 5% | 1 | Increased number of hairs | -39 | | | | |
| | | | Increased hair thickness (µm) | -0.007 | | | | |
| | | 2 | Increased number of hairs | | | | | 3 |
| | | | Increased hair thickness (µm) | | | | | 0.001 |
| | | 3 | Increased number of hairs | | | | | 4 |
| | | | Increased hair thickness (µm) | | | | | 0.002 |

| | | | | | | | | |
|---|---|---|---|---|---|---|---|---|
| Note: In the above table, each section without data represents that the number of hairs or the hair thickness was not measured at the corresponding time, and is the same in the following tables. | | | | | | | | |

The data shown in Table 4 are to determine the rapid onset of the effect of the composition for topical application of the present invention. That is, the data in Table 4 shows that both the number of hairs and the hair thickness significantly increase in almost all alopecia patients (nine patients administered with the compositions of the Example) 2 weeks to 4 weeks after the administration of the composition for topical application of the present invention. One patient showed decrease in the number of hairs after 4 weeks, but the number of hairs in this patient increased after 8 weeks.

Moreover, the data in Table 4 shows that both the number of hairs and hair thickness significantly increase in all alopecia patients (13 patients administered with the compositions of the Examples 1 to 3) 6 weeks to 8 weeks after the administration of the composition for topical application of the present invention.

As well known in the art, these effects of preventing hair loss prevention stimulating hair growth are very remarkable considering that these effects appear after at least 3 months in the conventional prescription (See the data in Comparative Example 1).

Specifically, according to the data in Table 4, the alopecia patients administered with the composition for topical application of Examples 1 to 3 of the present invention showed a significant increase in both the number of hairs and the hair thickness, compared to the patients treated with the conventional prescription of Comparative Example 1. That is, the alopecia patients administered with the composition for topical application of the present invention showed a more significant increase in both the number of hairs and the hair thickness after 2 weeks, and the effects increased gradually over time, resulting in a significant difference after 8 weeks compared to the alopecia patients treated with the prescription of Comparative Example 1.

The rapid onset of the effects of the above-described composition for topical application of the present invention suggests that the composition for topical application of the present invention can significantly improve the treatment compliance and satisfaction of patients. That is, in the case of the conventional prescription, the onset of the effects was very slow, and thus many patients abandoned the treatment. However, these problems can be significantly overcome by the use of the composition of the present invention.

Moreover, it can be said that the results of the clinical trials suggest the excellent effect of the composition for topical application of the present invention during exclusive use as well as the possibility of its use in combination with conventional oral medications.

That is, in the case of the conventional prescription, the effects of preventing hair loss and stimulating hair growth are not fully expressed until 3 months after the use, and thus the users' satisfaction is not high. This result reduces the treatment compliance of patients, leading the patients to abandon the treatment. However, in the case of the combination of the topical application of the present invention with the conventional oral medication, the effects of preventing hair loss and stimulating hair growth are rapidly expressed even at the beginning of treatment thanks to the characteristics of the composition for topical application of the present invention. Therefore, the treatment compliance and satisfaction of patients are significantly improved, and thus the possibility of abandoning the treatment is dramatically reduced.

**[Table 5]**

| No. | Drug administered | Example 1 | Example 2 | Example 3 | Example 4 | Comparative Example 1 | Comparative Example 3 |
|---|---|---|---|---|---|---|---|
| | | finasteride 0.001% + minoxidil 5% | finasteride 0.001% + minoxidil 2% | finasteride 0.002% + minoxidil 2% | finasteride 0.002% + minoxidil 5% | finasteride 1 mg+ minoxidil 5% | Minoxidil 5% |
| | Measuring time | 13 weeks | 13 weeks | 13 weeks | 13 weeks | 13 weeks | 13 weeks |
| 1 | Increased number of hairs | 15 | 29 | 32 | 16 | -26 | 10 |
| | Increased hair thickness (µm) | 0.009 | 0.006 | 0.035 | 0.019 | -0.015 | 0.005 |
| 2 | Increased number of hairs | 22 | 45 | 44 | 19 | -3 | 9 |
| | Increased hair thickness (µm) | 0.006 | 0 | 0.016 | 0.017 | 0.012 | 0.001 |
| 3 | Increased number of hairs | 9 | 21 | 29 | 32 | 1 | 11 |
| | Increased hair thickness (µm) | 0.007 | 0.011 | 0.006 | 0.035 | 0.002 | 0.01 |
| 4 | Increased number of hairs | 9 | 6 | 39 | 35 | 9 | 13 |
| | Increased hair thickness (µm) | 0.015 | 0.009 | 0.019 | 0.021 | 0.005 | 0.005 |
| 5 | Increased number of hairs | 19 | 5 | 9 | 24 | | -31 |
| | Increased hair thickness (µm) | 0.019 | 0.008 | 0.009 | 0.026 | | -0.016 |
| 6 | Increased number of hair | 26 | | 21 | 20 | | 3 |
| | Increased hair thickness (µm) | 0.021 | | 0.019 | 0.024 | | -0.005 |
| 7 | Increased number of hairs | | | 19 | 32 | | -12 |
| | Increased hair thickness (µm) | | | 0.009 | 0.026 | | -0.012 |
| 8 | Increased number of hairs | | | 26 | 19 | | -11 |
| | Increased hair thickness (µm) | | | 0.021 | 0.019 | | -0.019 |
| 9 | Increased number of hairs | | | | 20 | | -6 |
| | Increased hair thickness (µm) | | | | 0.033 | | 0.02 |
| 10 | Increased number of hairs | | | | 21 | | -11 |
| | Increased hair thickness (µm) | | | | 0.019 | | -0.016 |
| 11 | Increased number of hairs | | | | | | 10 |
| | Increased hair thickness (µm) | | | | | | 0.005 |
| 12 | Increased number of hairs | | | | | | -1 |
| | Increased hair thickness (µm) | | | | | | 0.001 |
| 13 | Increased number of hairs | | | | | | 14 |
| | Increased hair thickness (µm) | | | | | | 0.02 |
| 14 | Increased number of hairs | | | | | | 10 |
| | Increased hair thickness (µm) | | | | | | 0.002 |
| | increased number of hairs, | 16.6667 | 21.2 | 27.375 | 23.8 | -4.75 | 0.5714 |
| | average | | | | | | |
| | increased hair thickness (µm), average | 0.0128 | 0.0068 | 0.0168 | 0.0239 | 0.001 | 0.000071 |

**[Table 6]**

| Data of number of hairs, TTEST | | | |
|---|---|---|---|
| | Type | Data of Comparative Example 1 | Data of Comparative Example 3 |
| Data of Example 1 | FTEST | 0.130013191 | 0.166204493 |
| | TTEST | 0.01472931 | 0.011818468 |
| Data of Example 2 | FTEST | 0.894114552 | 0.458770619 |
| | TTEST | 0.046499133 | 0.012058313 |
| Data of Example 3 | FTEST | 0.475819066 | 0.69761868 |
| | TTEST | 0.001838518 | 0.000102744 |
| Data of Example 4 | FTEST | 0.051284979 | 0.048452534 |
| | TTEST | 0.000267943 | 0.000014812 |

| Data of hair thickness, TTEST | | | |
|---|---|---|---|
| | Type | Data of Comparative Example 1 | Data of Comparative Example 3 |
| Data of Example 1 | FTEST | 0.162697573 | 0.162697573 |
| | TTEST | 0.040829245 | 0.016450776 |
| Data of Example 2 | FTEST | 0.082456882 | 0.049214765 |
| | TTEST | 0.323896199 | 0.096803766 |
| Data of Example 3 | FTEST | 0.573873721 | 0.432576185 |
| | TTEST | 0.027277192 | 0.003679183 |
| Data of Example 4 | FTEST | 0.130017138 | 0.040734435 |
| | TTEST | 0.000342343 | 0.000004976 |

| | | | |
|---|---|---|---|
| * If p < 0.05, it is determined to have significance. | | | |

As shown in Table 5, the composition for topical application of Examples 1 to 4 of the present invention showed a significant increase in both the number of hairs and the hair thickness (µm) 13 weeks after the administration in most cases, compared to the conventional prescription of Comparative Examples 1 and 3. Moreover, it was found from the results of evaluation by TTEST shown in Table 6 that all data of hair thickness have significance except for the data of Example 2.

The results of the clinical trials are very important in that the composition for topical application of Examples 1 to 4 of the present invention exhibited more significant effects of preventing hair loss and stimulating hair growth even with the use of finasteride in an amount much smaller than that of the conventional prescription.

That is, in the case of oral administration of finasteride (1 mg/day), side effects such as hyposexuality, impotence, ejaculation disorder, etc. have been reported. Based on this, it can be said that the composition for topical application of the present invention significantly reduces the dose of finasteride and, at the same time, provides much better effects, thus providing an innovative way to solve the conventional side effects.

**[Table 7]**

| No. | Drug administered | Example 5 | | Example 6 | | Comparative Example 2 | |
|---|---|---|---|---|---|---|---|
| | | dutasteride 0.0002% +minoxidil 2% | | dutasteride 0.0005% +minoxidil 2% | | dutasteride 0.5mg +minoxidil 5% | |
| | Measuring time | 4 weeks | 8 weeks | 4 weeks | 8 weeks | 4 weeks | 8 Weeks |
| 1 | Increased number of hairs | 29 | 45 | 11 | 10 | 4 | 11 |
| | Increased | 0.019 | 0.018 | 0.301 | -0.286 | 0.002 | 0.006 |
| | hair thickness (µm) | | | | | | |
| 2 | Increased number of hairs | 10 | 26 | 19 | 16 | -16 | 5 |
| | Increased hair thickness (µm) | -0.003 | 0.008 | 0.028 | -0.001 | -0.011 | -0.003 |
| 3 | Increased number of hairs | 10 | 17 | 3 | 19 | 1 | 8 |
| | Increased hair thickness (µm) | 0.003 | 0.01 | 0.016 | 0.019 | 0.001 | 0.008 |
| 4 | Increased number of hairs | 13 | 22 | 2 | 22 | -1 | 6 |
| | Increased hair thickness (µm) | 0.006 | 0.005 | 0.01 | 0.011 | -0.022 | -0.01 |
| 5 | Increased number of hairs | 29 | 45 | 6 | 7 | | |
| | Increased hair thickness (µm) | 0.014 | 0.013 | 0.003 | 0.535 | | |
| 6 | Increased number of hairs | | | 6 | 21 | | |
| | Increased hair thickness (µm) | | | -0.005 | 0.003 | | |
| 7 | Increased number of hairs | | | | 16 | | |
| | Increased hair thickness (µm) | | | | 0.408 | | |
| | Increased | 18.2 | 31 | 7.8333 | 15.8571 | -3 | 7.5 |
| | number of hairs, average | | | | | | |
| | Increased hair thickness (µm), average | 0.0078 | 0.0108 | 0.0588 | 0.0984 | -0.0075 | 0.00025 |

**[Table 8]**

| Data of number of hairs, TTEST | | | |
|---|---|---|---|
| | Type | | Data of Comparative Example 2 (8 weeks) |
| Data of Example 5 | FTEST | | 0.024723978 |
| | TTEST | | 0.014726095 |
| Data of Example 6 | FTEST | | 0.248152042 |
| | TTEST | | 0.021595279 |

| Data of hair thickness, TTEST | | | |
|---|---|---|---|
| | Type | Data of Comparative Example 2(8 weeks) | |
| Data of Example 5 | FTEST | 0.685354662 | |
| | TTEST | 0.049411807 | |
| Data of Example 6 | FTEST | 0.000082551 | |
| | TTEST | 0.38806212 | |

| | | | |
|---|---|---|---|
| * If p < 0.05, it is determined to have significance. | | | |

As can be seen in Table 7, the compositions for topical application of Examples 5 to 6 of the present invention showed significant increases in the number of hairs and the hair thickness (µm) compared to the conventional prescription of Comparative Example 2. Moreover, it was found from these results of evaluation by TTEST shown in Table 8 that all data of hair thickness data have significance except for the data of Example 6.

The results of the clinical trials are very important in that the compositions for topical application of Examples 5 and 6 of the present invention exhibited more significant effects of preventing hair loss and stimulating hair growth even with the use of dutasteride in an amount much smaller than that of the conventional prescription.

That is, in the case of oral administration of dutasteride (0.5 mg/day), side effects such as hyposexuality, impotence, ejaculation disorder, etc. have been reported. Based on this, it can be said that the composition for topical application of the present invention significantly reduces the dose of dutasteride and, at the same time, provides much better effects, thus providing an innovative way to solve the conventional side effects.

Moreover, in the clinical trials, the changes in the number of hairs and hair thickness were measured 4 to 8 weeks after the administration, and the fact that composition for topical application of Examples 5 and 6 of the present invention exhibited more significant effects than the conventional prescription, indicating that the onset of the effects of the composition for topical application of the present invention is very rapid.

These results of the clinical trials indicate that the composition for topical application of the present invention can significantly improve the treatment compliance and satisfaction of patients due to the rapid effect.

That is, in the case of the conventional prescription, the onset of the effects was very slow, and thus many patients abandoned the treatment. However, these problems can be significantly improved by the use of the composition of the present invention.

Moreover, as mentioned in Examples 1 to 4, it can be said that the results of the clinical trials suggest the excellent effect of the composition for topical application of the present invention during exclusive use as well as the possibility of its use in combination with conventional oral medications.

### Test Example 2: Onset of effects and evaluation of side effects

**[Table 9]**

| | Exam. 1 | Exam. 2 | Exam. 3 | Exam. 4 | Exam. 5 | Exam. 6 | Comp. Exam. 1 | Comp. Exam. 2 | Comp. Exam. 3 |
|---|---|---|---|---|---|---|---|---|---|
| Number of patients in test group | 9 | 7 | 10 | 13 | 6 | 8 | 6 | 5 | 19 |
| Number of patients who abandoned the treatment due to no effect | 0 | 0 | 0 | 0 | 0 | 0 | 1 | 0 | 3 |
| Number of patients who Abandoned the treatment due to side-effect | 0 | 0 | 0 | 0 | 0 | 0 | 0 | 1 | 0 |

| | | | | | | | | | |
|---|---|---|---|---|---|---|---|---|---|
| Note: The above data were recorded after 32 weeks from the beginning of treatment. | | | | | | | | | |

As shown in Table 9, all of the hair loss patients administered with the composition for topical composition of Examples 1 to 6 of the present invention exhibited got the 100% effect, and there were no side effects except for two patients who experienced scalp pruritis temporarily.

On the contrary, in the case of Comparative Examples 1 to 3 of conventional prescription, 13% (4 patients) of the total patients (30 patients) of the test group abandoned the treatment due to no effect, and about 3.3% (1 patient) had side effects. These results of Comparative Examples 1 to 3 are considered to be consistent with the facts known about the side effects and the no effects.

## Claims

1. A pharmaceutical composition comprising a 5α-reductase inhibitor, and minoxidil or a pharmaceutically acceptable salt thereof, for use in the treatment of alopecia, wherein the composition is transdermally administered, and the daily dose of 5α-reductase inhibitor is 0.8 µg to 0.12 mg.

2. The pharmaceutical composition for use according to claim 1, wherein the daily dose of 5α-reductase inhibitor is 0.8 µg to 0.05 mg.

3. The pharmaceutical composition for use according to claim 1, wherein the daily dose of minoxidil. or a pharmaceutically acceptable salt thereof is 0.008 g to 0.12 g.

4. The pharmaceutical composition for use according to claim 1, wherein the 5α-reductase inhibitor is finasteride or a pharmaceutically acceptable salt thereof.

5. The pharmaceutical composition for use according to claim 1, wherein the 5α-reductase inhibitor is dutasteride or a pharmaceutically acceptable salt thereof.

6. The pharmaceutical composition for use according to claim 1, further comprising alfatradiol.

7. The pharmaceutical composition for use according to claim 1, further comprising a solvent containing a lower monoalcohol having 1 to 4 carbon atoms, a polyhydric alcohol, or water, and their mixtures.

8. The pharmaceutical composition for use according to claim 1, wherein the composition has a dosage form selected from the group consisting of hair tonic, scalp treatment, hair cream, general ointment, cosmetic water, powder, essence, pack, hair dye, shampoo, conditioner, lotion, gel, patch, and spray.

## Patentansprüche

1. Pharmazeutische Zusammensetzung, welche einen 5α-Reduktase-Inhibitor und Minoxidil oder ein pharmazeutisch akzeptierbares Salz davon umfasst, zur Verwendung in der Behandlung von Alopecia, wobei die Zusammensetzung transdermal verabreicht wird und die tägliche Dosis an 5α-Reduktase-Inhibitor 0,8 µg bis 0,12 mg beträgt.

2. Pharmazeutische Zusammensetzung zur Verwendung gemäß Anspruch 1, wobei die tägliche Dosis an 5α-Reduktase-Inhibitor 0,8 µg bis 0,05 mg beträgt.

3. Pharmazeutische Zusammensetzung zur Verwendung gemäß Anspruch 1, wobei die tägliche Dosis an Minoxidil oder einem pharmazeutisch akzeptierbaren Salz davon 0,008 g bis 0,12 g beträgt.

4. Pharmazeutische Zusammensetzung zur Verwendung gemäß Anspruch 1, wobei der 5α-Reduktase-Inhibitor Finasterid oder ein pharmazeutisch akzeptierbares Salz davon ist.

5. Pharmazeutische Zusammensetzung zur Verwendung gemäß Anspruch 1, wobei der 5α-Reduktase-Inhibitor Dutasterid oder ein pharmazeutische akzeptierbares Salz davon ist.

6. Pharmazeutische Zusammensetzung zur Verwendung gemäß Anspruch 1, welche des Weiteren Alfatradiol umfasst.

7. Pharmazeutische Zusammensetzung zur Verwendung gemäß Anspruch 1, welche des Weiteren ein Solvenz umfasst, das einen niederen Monoalkohol, der 1 bis 4 Kohlenstoffatome aufweist, einen polyhydrischen Alkohol oder Wasser und ihre Mischungen enthält.

8. Pharmazeutische Zusammensetzung zur Verwendung gemäß Anspruch 1, wobei die Zusammensetzung eine Dosierungsform aufweist, welche ausgewählt ist aus der Gruppe bestehend aus Haarwasser, Kopfhautbehandlung, Haarcreme, allgemeine Salbe, kosmetisches Wasser, Pulver, Essenz, Packung, Haarfärbemittel, Shampoo, Spülung, Lotion, Gel, Pflaster und Spray.

## Revendications

1. Composition pharmaceutique comprenant un inhibiteur de la 5α-réductase, et du minoxidil ou l'un de ses sels pharmaceutiquement acceptables, pour une utilisation dans le traitement de l'alopécie, dans laquelle la composition est administrée par voie transdermique, et la dose quotidienne de l'inhibiteur de la 5α-réductase étant de 0,8 µg à 0,12 mg.

2. Composition pharmaceutique pour une utilisation selon la revendication 1, dans laquelle la dose quotidienne de l'inhibiteur de la 5α-réductase est de 0,8 µg à 0,05 mg.

3. Composition pharmaceutique pour une utilisation selon la revendication 1, dans laquelle la dose quotidienne de minoxidil ou de l'un de ses sels pharmaceutiquement acceptables est de 0,008 g à 0,12 g.

4. Composition pharmaceutique pour une utilisation selon la revendication 1, dans laquelle l'inhibiteur de la 5α-réductase est le finastéride ou l'un de ses sels pharmaceutiquement acceptables.

5. Composition pharmaceutique pour une utilisation selon la revendication 1, dans laquelle l'inhibiteur de la 5α-réductase est le dutastéride ou l'un de ses sels pharmaceutiquement acceptables.

6. Composition pharmaceutique pour une utilisation selon la revendication 1, comprenant en outre de l'alfatradiol.

7. Composition pharmaceutique pour une utilisation selon la revendication 1, comprenant en outre un solvant contenant un monoalcool inférieur comportant 1 à 4 atomes de carbone, un polyol, ou de l'eau, et leurs mélanges.

8. Composition pharmaceutique pour une utilisation selon la revendication 1, la composition ayant une forme galénique choisie dans le groupe constitué de tonique capillaire, traitement du cuir chevelu, crème capillaire, pommade générale, eau cosmétique, poudre, essence, sachet, teinture capillaire, shampooing, après-shampooing, lotion, gel, patch, et pulvérisation.
